(19) Europäisches Patentamt / European Patent Office / Office européen des brevets

(11) **EP 4 317 088 A1**

(12) **EUROPEAN PATENT APPLICATION**

(43) Date of publication:
07.02.2024  Bulletin 2024/06

(21) Application number: 23187432.2

(22) Date of filing: 25.07.2023

(51) International Patent Classification (IPC):
**C02F 3/28** (2023.01)   **C12M 1/107** (2006.01)
**B65D 88/34** (2006.01)

(52) Cooperative Patent Classification (CPC):
**C02F 3/28;** C02F 2203/006; C12M 23/38;
C12M 23/56

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  05.08.2022  IT 202200016872

(71) Applicant: **Acqua & Sole S.r.l.**
**20124 Milano (IT)**

(72) Inventors:
• **NATTA, Francesco**
**27010 GIUSSAGO (PV) (IT)**
• **GIORDANO, Andrea**
**27010 VELLEZZO BELLINI (PV) (IT)**
• **BECCARELLI, Paolo**
**25050 PROVAGLIO D'ISEO (BS) (IT)**
• **MAFFEI, Roberto**
**25050 PROVAGLIO D'ISEO (BS) (IT)**

(74) Representative: **Bianchetti & Minoja with
Trevisan & Cuonzo IPS SRL
Via Plinio, 63
20129 Milano (IT)**

(54) **FLOATING COVER SYSTEM FOR TANKS FOR THE STORAGE OF ORGANIC MATRICES FOR SEASONAL USE**

(57)    The present invention relates to an active floating cover system for physically covering tanks and/or vessels for storing digestate and/or organic matrices for seasonal use or even only subject to the release of compounds which may cause olfactory nuisance and/or emissions of volatile compounds (such as, for example, $H_2S$, $NH_3$, $CH_4$) potentially harmful to the environment, with the simultaneous possibility of conveying and removing rainwater.

More particularly, the present invention relates to a floating cover system, made at least in part in plastic material, chemically inert to volatile compounds and to the matrices stored, for the covering of tanks used especially, but not exclusively, in the area of processes of anaerobic digestion of organic substrates, and in particular for tanks for the storage of the digestate obtained from the process. In this application, in fact, the aforementioned conditions, the organic nature of the material stored, the seasonal use of the same (which entails sudden fluctuations in level), the potential release of volatile compounds with a high odorous impact, as well as adverse weather conditions, occur simultaneously.

Fig. 1

**Description**

**FIELD OF THE INVENTION**

The present invention relates to a floating cover system for the covering of tanks or vessels for the storage of digestate and/or organic matrices and/or other pumpable matrices for seasonal use or even only subject to the release of compounds that may cause olfactory nuisance or be potentially harmful to the environment.

More particularly, the present invention relates to a floating cover system, made at least in part in plastic material for the covering of tanks used especially, but not exclusively, in the area of processes of anaerobic digestion of organic substrates, and in particular for tanks for the storage of the digestate obtained from the process or for ingestates, slurry or other pumpable matrices. In this application, in fact, the aforementioned conditions, the organic nature of the stored material, the seasonal use of the same, the potential release of volatile compounds with a high odorous impact and/or potentially harmful to the environment, as well as adverse weather conditions, take place simultaneously.

The cover system according to the present invention allows a plurality of advantages to be obtained, especially in the covering of large tanks, but not only, serving as an alternative to known cover systems that have various disadvantages, whether of the fixed or floating type.

**STATE OF THE ART**

The use of storage tanks for the containment of digestate and slurry for agricultural use, as well as pumpable matrices, is known from the state of the art.

More specifically, in the area of processes of anaerobic digestion of organic substrates, it is common to store the product (e.g. digestate or ingestates or other pumpable matrices) obtained from the process and intended for agricultural use in storage tanks, in that, in general, the periods of agricultural use of the digestate are conditioned by the inherent seasonality of agriculture, and consequently are typically concentrated in two time windows, spring and autumn, while the feeding of storage from the digestion process takes place in an almost constant and continuous manner throughout the entire year. The same need may also arise in sectors other than agriculture characterised by seasonal activities.

This entails, on the one hand, the need to provide tanks with large storage volumes, and therefore of large diameter, and, on the other hand, the fact that the emptying of the tank takes place very quickly, being concentrated in about 3-4 months per year, with much higher flow rates with respect to those of feeding the tank itself.

Typically, it can be assumed that, in the course of a year, a volume equal to double the geometric capacity of the storage tank itself passes through it.

Large storage tank diameter (typically greater than 50 m), the need to store organic matrices, but not only these, which may determine the release of volatile compounds with potential odorous impact and/or potentially harmful to the environment and/or viscous thixotropic fluids, and the need to operate significant and sudden variations in the level of fluid stored, with the concomitant need to collect and convey rainwater and volatile compounds that are produced by the matrices stored, are typical conditions of use of the cover system according to the present invention.

In fact, in such conditions of use inside the storage tank very particular fluid-dynamic conditions occur, both due to the feeding and emptying conditions described above, and due to the particular rheological characteristics of the digestate, which is characterised by high viscosity values that give it a plastic type behaviour, with a constitutive bond (viscosity - shear rate) that can be expressed by means of a Hershel-Bulkley type law:

$$\mu_{\text{eff}} = \begin{cases} \mu_0, & |\dot{\gamma}| \leq \dot{\gamma}_0 \\ k|\dot{\gamma}|^{n-1} + \tau_0|\dot{\gamma}|^{-1}, & |\dot{\gamma}| \geq \dot{\gamma}_0 \end{cases}$$

Where $\tau0$ represents the sterile stress; k is the consistency index; $\gamma0$ is the shear rate beyond which the viscosity decreases; $\mu0$ is the viscosity value corresponding to $\dot{\gamma}_0$

This plastic behaviour is amplified in the case of digestates from anaerobic digestion of sewage sludge and of OFMSW as the dry matter content increases.

The high viscosity of the product, in addition to the typical hydrodynamic conditions that occur inside these tanks (as mentioned, high discharge rates concentrated in a few periods of the year), means that greater tangential stresses are transmitted to the cover than would be the case if the product were water or a petroleum derivative.

Moreover, and this is not an aspect of secondary importance, almost all the stresses are concentrated on a small portion of the cover, at the discharge outlets.

Last but not least, the fermentable residue connected to the stored matrices makes it necessary, also for safety reasons, for a cover to be present that is capable of avoiding, under any operating conditions, even extraordinary ones, accumulation inside the tank of large quantities of biogas as well as the containment of any malodorous emissions and/or volatile compounds potentially harmful to the environment.

Although the fields of application of the present invention are many, it has been specifically researched and optimised to form the covering of storage tanks used in the area of processes of anaerobic digestion of organic substrates, and in particular for tanks for the storage of the digestate obtained from the process.

Typically, cover systems for tanks and/or vessels for the storage of digestate intended for agricultural use are of two types: fixed or floating and made in concrete or metal according to the dimensions of the tank.

Both of these known cover systems have disadvantages.

More particularly, fixed covers of tanks or tanks with a large diameter, suitable for the storage of organic matrices with fermentable residue with a high odorous impact, and/or thixotropic viscous fluids, under conditions that lead to significant and sudden variations in the level of fluid stored, have the following disadvantages.

When the level of the fluid stored is low, large volumes of gas, including potentially explosive biogas, are formed in the headspace of the tank, i.e. in the volume comprised between the cover and the surface of the fluid stored. In addition, there is generally significant formation of foam on the surface of the liquid stored. In the case of fixed covers made of concrete, moreover, temperature differences between the internal and external environment favour the formation of cracks, with consequent gas leakages.

The high viscosity of the fluid stored, in addition to the typical hydrodynamic conditions that are created inside these storage tanks (high discharge flow rates concentrated in a few periods of the year), instead leads to problems in the case of floating cover. In this case, in fact, under the operating conditions indicated above, greater tangential stresses are transmitted to the cover than those that would occur if the product were water or a petroleum derivative and, above all, almost all the stresses are concentrated on a reduced portion of the cover, at the discharge outlets.

In the case of metal floating covers, those used for the storage of petroleum products are typically made in non-corrosion-resistant structural steel. The cover is obtained by simply welding together flat sheets that are, in use, in contact with the product and which, in the periphery zones (i.e. at the shell), rest on a toroid with trapezoidal section that acts as floating tank.

The disadvantage of this solution is represented by the fact that the welding of the sheets, on the side in contact with the product, is poorly protected from the corrosive agents present in the residual biogas and/or other corrosive compounds (also present in traces) that could be formed by the product during the storage period, and even a good coating would entail significant maintenance costs with difficulty of access to confined spaces for restoring the coating.

Finally, the headspace of storage tanks of the known type can contain biogas with strong corrosive capacities, in that it mainly contains ammonia, sulphides and terpenes that originate from the anaerobic degradation of the organic matter still present in the digestate.

The solution of making such a structure in stainless steel would make it particularly expensive.

In addition, the removal of rainwater involves discharge by means of the use of an articulated pipe that is housed inside the product itself. While this solution can be suitable in the case of petroleum products, it cannot be so in the case of products digested from organic material (chemically much more aggressive in terms of corrosion) as it would make the system unreliable and could be subject to failure of the joints.

In the area of a process involving anaerobic degradation, even residual, of organic matter, as well as for covers of many other pumpable substances, the installation of a floating cover system of the type proposed makes it possible to:

- reduce the volume of gas storage, a factor that instead constitutes the main limitation of fixed-type covers;
- recover biogas;
- avoid uncontrolled emissions of methane and ammonia into the atmosphere and, more generally, of volatile and odorous compounds;
- regulate rainwater, avoiding entry thereof inside the tank;
- safely access the cover for monitoring and maintenance;
- use materials that are compatible with both the stored matrices and the volatile compounds that are released during storage.

Floating covers also make it possible to cope with variations in the level of the liquid in tanks for storage of digestate, avoiding the formation/presence of large volumes of gas in the headspace (i.e. that present between the cover and the level of the stored liquid), which can also become contaminated with infiltration of air from the outside. Air infiltrations take place in the case of vacuums created by the abrupt lowering of the level of the liquid when emptying the tank or vessel.

The presence of oxygen, fed into the tank from the outside air, increases the risk of explosions that take place in the case of the simultaneous presence of combustible gas, oxygen (in certain proportions) and an igniter.

In the case of tanks for the storage of organic matrices intended for agricultural use as fertilisers, therefore with the timing of emptying of the tank linked, as mentioned above, to seasonality, the levels are highly variable, unlike those, always practically constant, present in digesters. The possibility of air infiltration inside the headspace is therefore one of the main risks in the operation of such structures. To overcome this problem, costly systems are usually implemented to compensate for overpressure by introducing nitrogen into the headspace, systems which are not necessary by implementing the present invention.

## SUMMARY OF THE INVENTION

In light of the foregoing, it is the task of the present invention to provide a cover system for storage tanks or vessels, in particular tanks of large dimensions, typically but not exclusively having a diameter even greater than 50 m, and suitable for storing pumpable matrices, above all organic with fermentable residue and/or thixotropic viscous fluids and/or substrates with potential release of noxious odorous compounds and volatile compounds potentially harmful to the environment, wherein the level of the stored fluid may undergo significant and sudden variations, capable of overcoming the disadvantages that afflict fixed or floating cover systems known from the state of the art.

Within this task, it is the object of the present invention to provide a floating cover system for storage tanks that allows limiting of the formation of the crust in the headspace and which guarantees on the one hand the accumulation of very small volumes of biogas in the headspace reducing the risk of explosiveness, and on the other hand the emission of malodorous compounds in any operating condition by recovering them and if possible exploiting them.

It is also the object of the present invention to provide a floating cover system for storage tanks or vessels that has intrinsic safety with respect to a possibility of collapse: the rainwater is removed from the cover by means of a specific dedicated system, but in the event of unforeseen operating conditions it flows into the tank without compromising functionality thereof, even in the event of particularly heavy rainfall.

Not least, it is an object of the present invention to provide a floating cover system for storage tanks which is suitable for use within a wide range of temperatures of the digestate.

Furthermore, it is an object of the present invention to provide a floating cover system for storage tanks that guarantees accessibility of the operator to the tank in safety in the case of ordinary and extraordinary maintenance operations.

It is a further object of the invention to provide a floating cover system for storage tanks that allows limiting of any uncontrolled gas and/or malodorous emissions, thanks to the ability to keep the system under vacuum, and by the fact that the volume of gas accumulation is limited to the slots present between two adjacent elements of the floating cover.

It is a further object of the invention to provide a floating cover system of a modular type, which allows scalability for tanks or vessels of any diameter and shape.

Furthermore, it is an object of the present invention to provide a floating cover system that can also be installed with tanks or vessels in operation or, vice versa, that allows easy removal and subsequent restoration in the case whereby reclamation and/or structural work needs to be carried out inside the tank.

Last but not least, it is an object of the present invention to provide a modular floating cover system that can also be only partially disassembled by removing portions of variable sizes of cover to allow or facilitate maintenance operations.

These and other objects, which will be made clearer following the detailed description of a preferred but not exclusive embodiment of the present invention, are achieved by a floating cover system for storage tanks in accordance with the appended claim 1.

Further features of the closure system are the subject of the dependent claims.

## LIST OF DRAWINGS

Further features and advantages will be made clearer by the following non-limiting illustrative description of a preferred embodiment of the present invention which will be given herein below with the aid of the accompanying drawings in which:

Figures 1 and 2 show an overall view of a portion of the floating cover system according to the present invention;
Figure 3 shows a detail of how in the cover system according to the present invention the tensioning system ensures tensioning of the cover membrane to a metal tubular element;
Figures 4 and 5 show the caissons that enable attachment of the floating raft, not shown in these drawings, and of the tensioning system;
Figures 6, 7 and 8 show perspective views of the support elements in the form of metal caissons;

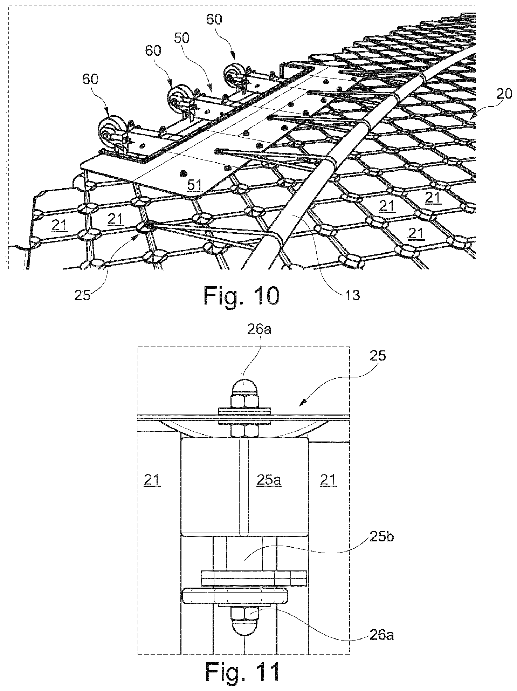

Figures 9 and 10 show the floating raft assembled to the metal caissons;

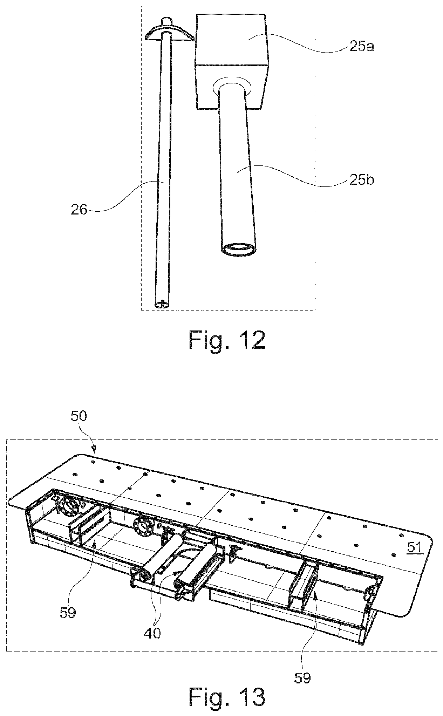

Figures 11 and 12 show in detail a preferred embodiment of the rigid connector according to the present invention;
Figures 13 and 14 show further details of the caisson, in particular, the low-friction rollers are visible, suitable for adequately constraining the cover preventing rotation thereof and, in Figure 14, the vertical anti-rotation pole;

Figure 15 illustrates in detail the presence of metal plates and gaskets that guarantee the hermetic seal of the connection of the cover to the caissons thanks to the tightness exerted by the fixing screws;

Figure 16 shows in detail, assembled and in a exploded-view, the configuration of the buffer elements associated with the caisson which enable the forces transmitted between the caisson and the shell of the tank in the XY plane to be absorbed;

Figures 17 and 18 show the positioning of the buffer elements, the low-friction rollers and the anti-rotation pole;

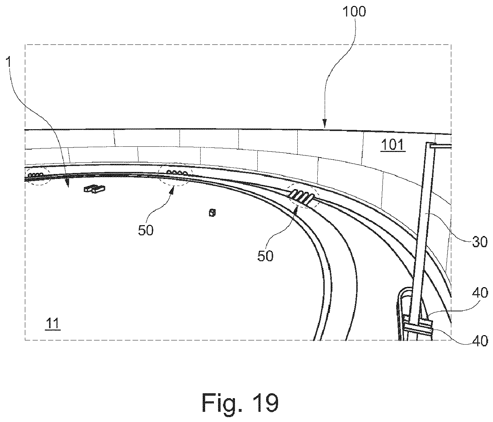

Figure 19 shows by way of example an overall view in which the position of the caissons, of the low-friction rollers and of the anti-rotation pole can be understood in relation to the cover as a whole;

Figure 20 illustrates a view from above of the floating cover system according to the present invention in which the flap, still to be turned up, can be seen. It then, once turned up (Fig. 18), goes to complete the cover;

Figure 21 illustrates a preferred embodiment of the water catchment system that comprises a circular water accumulation area;

Figures 22 and 23 illustrate the system for capturing biogas or any malodorous emissions deriving from the stored material according to the present invention, comprising an extraction pipe and a drum for winding said pipe.

## DETAILED DESCRIPTION OF THE INVENTION

With particular reference to the accompanying drawings, the floating cover system **1** for storage tanks **100** according to the present invention comprises a cover element in turn comprising a cover layer **10** comprising a membrane **11** in waterproof fabric, suitable for containing the biogas that may be generated during the periods of storage of the digestate.

Said membrane **11** in waterproof fabric performs the function of physical separation between the external environment and the digestate, preventing rainwater from entering the storage tank **100** and at the same time preventing biogas and/or malodorous emissions from escaping therefrom.

Furthermore, the membrane protects the underlying structure from the weather in general, therefore not only from rainwater but also from UV rays for example.

Advantageously, the cover layer **10** further comprises a tensioning system **12** configured to distend the fabric membrane **11,** avoid wrinkles and ensure an adequate level of pre-tensioning of the membrane itself (necessary in the case of strong wind gusts, internal gas pressure and similar circumstances).

The tensioning system **12** preferably comprises a perimeter metal tube **13** of a diameter appropriate to the force required for the tensioning and at the height required to contain the water. The metal tube **13** is in turn advantageously divided into sections with a bayonet coupling capable of adapting, without compromising the behaviour thereof, to the increasing perimeter development during the tensioning phases.

Furthermore, the floating cover system **1** according to the present invention further comprises a layer of reinforced nonwoven fabric **14** which is resting, and not welded, on said fabric membrane **11,** and a flap **15** capable of adapting to the edge of the membrane **11** and ballasted in order to guarantee, by means of immersion, the airtightness of the covering **10** by means of a "hydraulic trap". The ballasting is preferably made by means of the insertion of sand in special pockets formed in the flap **15** in a quantity appropriate to the level of operating pressure/vacuum of the system that it exerts, as a consequence, on the cover layer **10.**

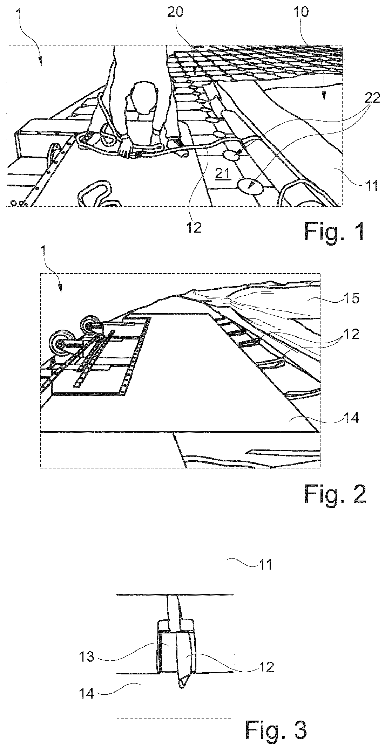

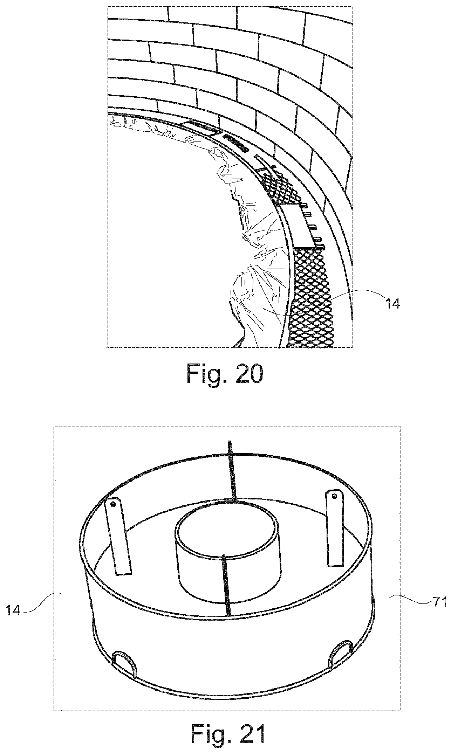

With particular reference to the accompanying drawings, in Figure 2 the flap is shown turned up, so that the layer of reinforced nonwoven fabric **14** resting on the fabric membrane **11** is visible, while in Figure 20 the flap **15** is shown not turned up, so that it covers the entire cover.

The cover layer **10** is also advantageously provided with reinforcements welded at the perimeter anchorage to support elements **50** of the cover **10** configured to guide the cover layer **10** in its movements along the vertical direction Z, at the same time limiting the movements along the horizontal directions X and Y

More particularly, advantageously said support elements **50** of the cover **10** are made up of metal elements **50.**

Layers of a reinforced nonwoven fabric **14** are advantageously positioned between the upper surface of the raft **20** overall formed by the modular floating elements **21,** and the membrane fabric **11,** in order to protect the upper sheet (edges of the support elements, the edge of the raft, the tensioning plates of the sheet, etc.).

Alternatively, the fabric cover layer **11** stretched inside the tensioning ring **13** can also be made in stainless steel sheet metal welded in place to increase durability thereof, reducing routine maintenance work to only the flap portion **15** in fabric.

The floating cover system **1** for storage tanks according to the present invention further comprises a floating raft **20** formed by a plurality of modular floating elements **21** connected one to the other at restraining joints **22** so as to form the tank cover.

When said floating raft **20** made up of a plurality of modular floating elements **21** is resting on the surface of the product stored inside the tank, at said joint areas **22** the passage of any biogas and/or any malodorous emissions that may be generated during storage is enabled.

Said floating raft **20,** which is part of said covering element and forms the actual "roof" of the tank, is provided with rigid connectors **25** having the function of efficiently transferring the concentrated loads to the floating raft **20.** The connectors are made up of a box element **25a** of suitable size to occupy the free space between the modular floats **21.** The box element is completed by a tubular element **25b** of adequate size to fit into the point of joining of the modular floats **21** and internally hollow in order to accommodate a threaded bar **26** for tightening of the joint by screwing one or more bolts **26a.** The connector is used for structural connection to other elements of the floating cover system **1** according to the invention, such as in particular to the caissons **50** and to the tensioning system **12** of the sheet in waterproof fabric **11.**

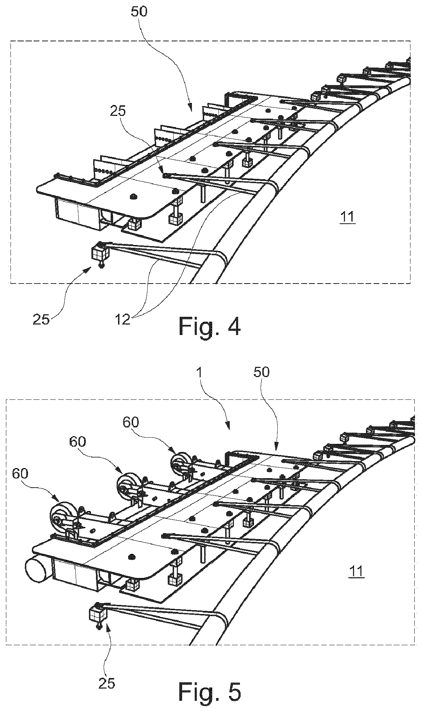

The rigid connectors **25** are placed along the entire perimeter of the cover at variable centre distance and allow the connection of the floating raft **20** to the support elements **50,** as can be seen for example in Figures 4 and 5, and the tensioning of the membrane **11** which, by means of the perimeter metal tube **13** and the tensioning system **12,** is fixed to the rigid connectors **25** placed at the floating raft and/or the support elements **50.**

Providing modular elements **21** that can be connected one to the other makes it possible to make the entire system modular, allowing the geometry of the cover to be adapted to storage tanks or vessels of various sizes and shapes (circular, rectangular, elliptical, etc.).

The size and thickness of the modular elements **21** and their joining system are a function of the stresses transmitted to the floating raft **20** by the digestate. It is essential that the resistance in the plane is adequate for the tensile, compressive and shear stresses generated by the forces of contact with the digestate. It is also essential that the floating raft is adequately flexible out of the plane so that it can adapt to the unevenness of the surface of the digestate during the phases of entry and exit of the same to/from the tank/vessel. Out-of-plane flexibility also makes it possible to create valley points in the cover layer, making it possible for rainwater to drain off. The out-of-plane flexibility of the floating raft also allows the cover to lie on an uneven bottom with obstacles (the liquid inlet, suction and recirculation pipes) and to adapt to any abnormal waves of the liquid in the event of earthquakes or strong winds.

The floating cover system **1** for storage tanks/vessels according to the present invention further comprises, fixed to the inner wall of the tank preferably with an interlocking type joint at its ends, one or more anti-rotation elements **30,** in the example illustrated in the drawings made up of a vertical pole, suitable for limiting at least partially the movements of the cover element **10, 20** with respect to the tank **100.** The anti-rotation pole **30** advantageously placed at a certain distance from the shell **101** of the tank, according to what can be seen in the accompanying drawings, allows a part of the stresses transmitted from the cover to the shell **101** in the plane **XY** to be absorbed. At the same time, the roller system, which will be described in greater detail here below, allows displacements in the vertical direction **Z** of the cover. The anti-rotation pole **30** represents the fixed point of the floating cover, and the cover is restrained thereto by means of the caissons **50,** and in particular the rollers **40,** as will be described here below.

The interlocking joint enables part of the stresses developed by the floating cover during normal operation to be transferred to the shell, i.e. to the inner wall of the tank, preventing the floating raft from rotating around the centre of the tank.

The floating cover system **1** for storage tanks/vessels according to the present invention further comprises, acting between the anti-rotation pole **30** and the floating cover layer **10,** a system of low-friction rollers **40** suitable for adequately constraining the cover by preventing rotation thereof in the plane **XY** without damaging either the anti-rotation pole **30** or the caisson **50.**

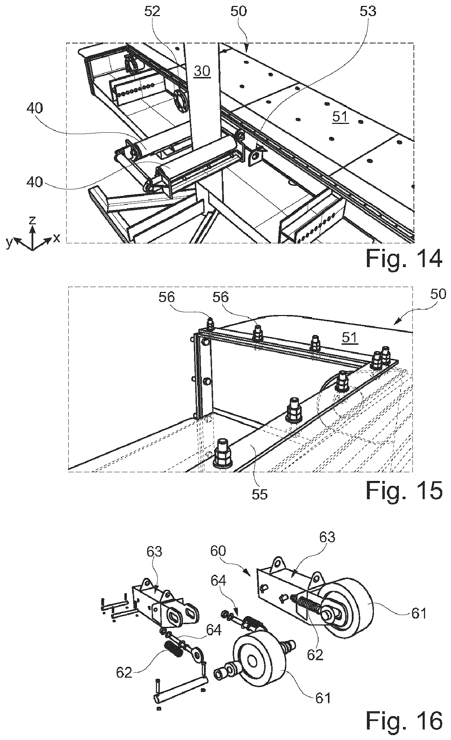

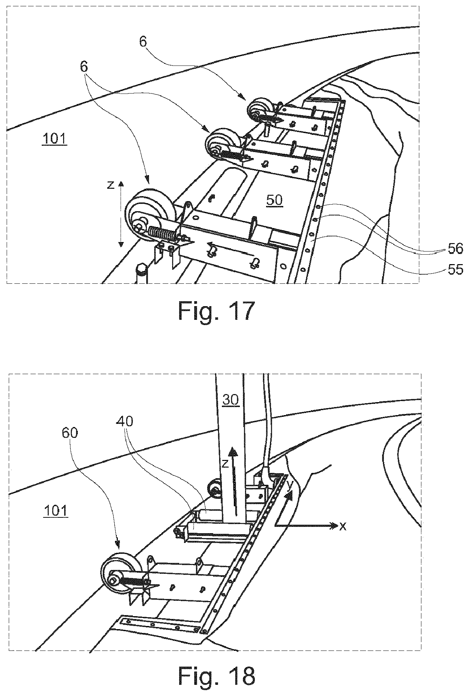

With particular reference to Figures 14 and 18, the rollers **40** constrain the horizontal movement in a direction, denoted by **Y** in the Cartesian reference of Figure 14 of the horizontal plane **XY,** preventing, in fact, the rotation of the floating cover layer **10** that could be caused by the stresses deriving from the vortical movements that may occur in the stored fluid.

In the direction denoted by **X** in Figures 14 and 18, orthogonal to the constrained **Y** one, the roller system ensures a stroke with length defined in the design phase in order to manage the movements and deformations of the cover in that direction during operation. The rollers **40** instead allow movement in the vertical direction, denoted by **Z** in the abovementioned drawings.

Advantageously, the contact surfaces of rollers **40** are made in Teflon or a suitable material to limit friction and wear over time as well as the risk of sparks (which could be a trigger for possible explosions). The core of the rollers **40** is preferably made with metal profiles to guarantee the required mechanical strength.

The floating cover system **1** for storage tanks according to the present invention further comprises one or more metal caissons **50** to which the floating raft **20** is connected and which perform the function of an interface with the inner wall of the tank.

On said metal caissons **50,** in fact, buffer elements **60** are installed which allow the floating cover **1** to be kept at a sufficient distance from the inner wall of the tank: during the operations of emptying and/or filling, in fact the cover would tend to impact on the wall itself, damaging the wall, any layers of protective coating, the flap of the cover layer and the floating modules. The buffer elements **60** allow the forces on the **XY** plane to be absorbed. They are provided with wheels **61** to allow movements along the vertical **Z.** The wheel **61** is mounted on special wheel holders, or rather buffer holders, **59,** made with perforated metal flanges **52** which allow the buffers **60** of the wheels **61** to be mounted and adjusted.

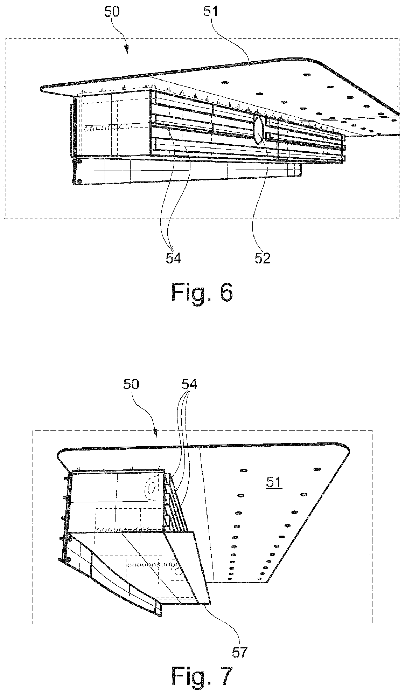

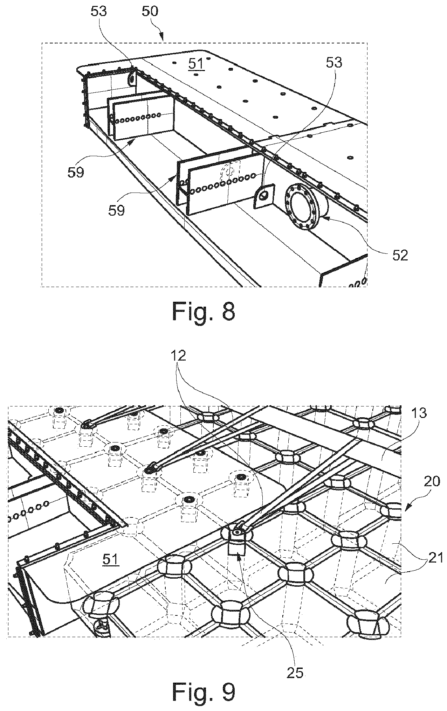

With particular reference to Figures 6, 7 and 8, each metal caisson **50** comprises a perforated upper plate **51** to which is restrained the floating raft **20** made with the floating modules **21** by means of rigid connectors.

Each of said support elements **50** may have one or more flanges **52** for aspiration of the gas and a system of spacers **54** to facilitate the drainage of the gas at the vertical surface of contact between the wall of the tank and the floating raft. The same spacers **54** also act as ribs suitable for improving the mechanical resistance to the stresses exerted by the buffer elements **60** installed on said support elements **50.**

Advantageously the support elements **50** are provided with hooks for lifting **53** and movement during phases of assembly and maintenance, with a horizontal flange **57** for C-clamping the floating raft, a vertical flange **58** appropriately dimensioned to ensure a "hydraulic trap" in line with that guaranteed by the cover layer (which is interrupted at the caissons).

The support elements **50** perform the function of transferring to the buffers with wheels 60 (and subsequently to the layer **101)** the actions due to the stresses generated by the movement of the liquid in contact with the floating cover **1.**

The buffers with wheels **60,** in fact, cannot be connected directly to the modules in plastic material **21** because the maximum force for which they were designed would damage the single module **21** and it must therefore be distributed over several modules **21,** a function performed by the caisson.

Moreover, the support elements **50** guarantee a stable and secure landing point during maintenance operations. For example, the support elements **50** can also constitute a stable attachment point for the rails of a ladder that connects a balcony provided on the tank to the floating raft **20.**

Last but not least, the support elements **50** can be used to connect instrumentation and accessories that otherwise could not be connected directly to the cover fabric **10** or to the plastic material modules **21,** such as the aspiration flanges **52** of the gas/malodorous emissions, low-friction rollers **40,** sensors and data acquisition units and so on, as well as ensuring also the anti-rotation system of the cover itself.

The interface between the support elements **50** and the cover layer **10** is formed by means of a mechanical fastening system with metal plates **55** and gaskets that guarantee the airtight seal thanks to the tightness exercised by the screws **56** (replaceable in the case of seizure).

As mentioned, a plurality of buffer elements **60** provided with wheels **61** and shock absorbers **62** are interposed between the shell and the support elements **50,** the latter being then connected to the floating raft **20.** Said shock absorbers **62** are suitable for counteracting thrust forces that are exchanged between the buffer element **60** of said support element **50** and the inner wall of the tank/vessel **100.**

With reference to the accompanying drawings, when the buffer elements **60** are configured as described, they protrude radially from said caissons **50,** said shock absorbers **62** acting along the radial direction.

Advantageously, said wheels **61** of said buffer elements **60** are provided with a rubber lining to facilitate vertical movements of the floating raft **20** that take place during filling and emptying, so as to limit friction and wear damage to the shell **101** and to any protective coatings.

Said buffer elements **60** can also advantageously comprise at least one wheel support pin **61** for transferring the load to the wheel holder assembly **63** and inserts in Teflon to limit friction between the metal components and eliminate the risk of sparks.

A threaded bar system **64** can advantageously be provided for adjusting the position of the wheel **61** with respect to the wheel holder **63.**

The spring **62** allows the adjustment of the 'preload' level and improvement in force distribution. Thanks to the action of the springs **62,** the wheel **61** orientates so as to work in adherence to the shell. Thanks to the action of the springs **62** it is also possible to improve the distribution of forces between the buffers **60** of the same caisson **50.**

A variable number of buffers **60** on each caisson **50** can be provided as a function of the force that has to be transferred from the raft **20** to the vertical wall of the vessel. There can be, by way of example, two, three or five buffer elements **60** mounted on each support element **50.**

The forces mainly take shape during the periods of rapid filling and emptying of the tank/vessel and vary as a function of the viscosity of the material and of the level of digestate in the same.

Where necessary, the buffer element **60** or wheel assembly may be provided with flanges for the connection of additional floats **21** aimed at improving the flotation of the support element **50.**

In accordance with a preferred embodiment of the floating cover system **1** for storage tanks/vessels according to the present invention it advantageously comprises a water catchment system formed by means of localised lowerings **71** of the cover and the consequent formation of a water catchment and drainage cone.

The shape of these water accumulation areas **71** is preferably circular, according to what is shown by way of example in Figure 21, and of adequate diameter, indicatively around 2 metres in diameter according to the properties of the fabric and the conditions of use of the cover, so as not to tear the fabric in the case of swelling of the cover layer due to internal pressure or action of the wind.

The localised lowering **71** constitutes a housing for the suction pump and must have a smooth contact surface that does not damage the cover layer and a suitable geometry, for example, the circular one shown in Figure 21, with openings capable of conveying water to the pump aspiration point. It must also be easy to inspect (e.g. to facilitate the removal of leaves or of any dirt that may impede the normal operation of the pump).

At the point of contact with the accumulation areas **71,** the cover layer is protected with additional layers of fabric and nonwoven fabric.

Rainwater management is assisted by the tubular metal ring **13** for tensioning the membrane, which also has the function of constituting a barrier to the normal outflow of water along the edges which makes it converge in the area of action of the suction pump.

The suction pump must have a system of feeding and outflow capable of adapting (by adjusting the length thereof) to the variable level of the liquid.

In accordance with a preferred embodiment of the floating cover system **1** for storage tanks/vessels according to the present invention, it further comprises a system for capturing biogas or possible malodorous emissions deriving from the stored material.

Advantageously, said biogas/emissions capturing system comprises an extraction pipe **80** connected to the support element **50** by means of an appropriate flange **52** at the drainage system of the support element **50** itself.

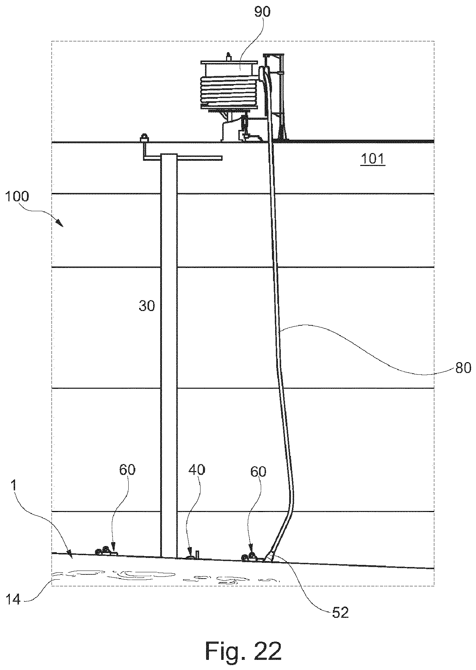

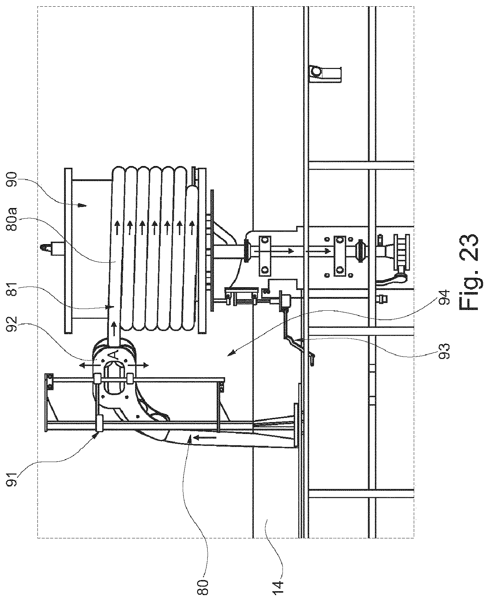

Advantageously, as shown by way of example in Figures 22 and 23, said extraction pipe **80** is flexible and of adjustable length so as to be adapted to the variable level of the liquid contained in the tank. Further, said extraction pipe **80** is advantageously provided, for the adjustment of its length, with a drum **90** for the winding of said pipe, made so as to have a single high point, denoted by reference numeral **81** in Figure 23, so as to allow the discharge of any condensation that may form in the course of the aspiration of the biogas/emissions. The length of the unwound fraction of said extraction pipe **80** can be adapted to the operating conditions by means of manual or automatic systems. The maximum length is reached, according to what can be seen by way of example in Figure 22, when the raft **20** is at the lowest point of the storage tank **100,** the minimum length is reached when the raft **20** is at the highest floating point. The maximum length of the extraction pipe **80** is therefore equal to the height of the tank, while the minimum length is equal to the difference between the height of the tank and the level of floating of the raft **20** when the tank is filled to its maximum level.

As mentioned, in order to guarantee the outflow of any condensation, and therefore the full functionality of the biogas/emissions extraction pipe **80,** a drum system **90** has been specifically implemented for the winding of the extraction pipe **80** suitable for simultaneously adjusting the length of this pipe **80** and the height of its upper coil **80a,** rolled on the drum itself, as the level of digestate contained in the storage tank **100** and, therefore, of the cover **1** according to the present invention varies.

More particularly, Figure 23 shows the support device **91** of the extraction pipe **80** is suitable for supporting and moving the upper end of this pipe **80** in the winding and unwinding operations on and from the drum **90.** This device **91** is installed externally to the storage tank **100.**

Said support device **91** for the extraction pipe comprises a vertically movable head **92** apt to support the upper end of said extraction pipe **80** and movable along the vertical axis Z by means of an adjustment system of the height of said head **92,** in turn, comprising a crank **93** connected to motion transmission means **94** that act at the same time on both an adjustment mechanism of the height of the head **92** and on the rotation of the drum **90.** Thanks to this support device **91** of the extraction pipe **80,** the operator can adjust the height of the high point **81** by moving upwards or downwards said head **92** as a function of the position of the last coil, the highest one **80a,** wound on the drum **90,** in order, as said, to ensure the outflow of any condensation.

It has thus been shown how the floating cover system for storage tanks/vessels according to the present invention achieves the intended objects and task.

The cover system according to the present invention may undergo modifications and variations, all of which fall within the scope of the invention as defined by the appended claims.

**Claims**

1. Floating cover system (1) for the covering of storage tanks or vessels (100), in particular for the storage of digestate, **characterised in that** it comprises a cover element (10, 20) comprising a floating raft (20), in turn comprising a plurality of modular floating elements (21) restrained one to the other at joining points (22) and configured to float by resting on the free surface of the digestate product contained in the tank/vessel.

2. Floating cover system (1) according to the preceding claim, **characterised in that** said cover element (10, 20) further comprises a cover layer (10) placed above said floating raft (20) and comprising at least one portion in waterproof fabric (11), said cover system (1) also comprising one or more support elements (50) of said cover element whereto said floating raft (20) and said cover layer (10) are reversibly connected.

3.  Floating cover system (1) according to the preceding claim, **characterised in that** said one or more support elements (50) of said cover element (10, 20) are made up of substantially box elements, preferably in metal material, to which said cover layer (10) is fixed by means of a hermetic mechanical fastening system, preferably comprising a plurality of metal plates (55).

4.  Floating cover system (1) according to one or more of the preceding claims, **characterised in that** said support elements (50) comprise buffer elements (60) configured to come into contact with the inner wall of the tank or vessel (100) maintaining said cover element (10, 20) at a predetermined distance from said wall, in such a way as to allow movement in a vertical direction of the cover element (10, 20) without friction against said wall.

5.  Floating cover system (1) according to the preceding claim, **characterised in that** said buffer elements (60) comprise one or more wheels (61) mounted on wheel holder elements (59) restrained to said support element (50), said wheel holder elements (59) further supporting one or more shock absorbers (62) suitable for contrasting thrust forces that are exchanged between the buffer element (60) and the inner wall of the tank (100), said one or more shock absorbers (62) preferably comprising a spring suitable for allowing adjustment of the preload and optimising the homogeneous distribution of the thrust forces.

6.  Floating cover system (1) according to the preceding claim, **characterised in that** said buffer elements (60) further comprise a threaded bar system (64) for adjusting the position of said wheel (61) with respect to said wheel holder (63).

7.  Floating cover system (1) according to one or more of the preceding claims, **characterised in that** it further comprises one or more anti-rotation elements (30), suitable for at least partially limiting the movements of the cover element (10, 20) with respect to the tank (100).

8.  Floating cover system (1) according to the preceding claim, **characterised in that** said support elements (50) further comprise a system of rollers (40) suitable for limiting the movements of the cover element (10, 20) in the horizontal plane (XY) preventing rotation thereof, avoiding damage to the anti-rotation pole (30) and the support element (50).

9.  Floating cover system (1) according to one or more of the preceding claims, **characterised in that** said cover layer (10) further comprises a tensioning system (12) in turn comprising a perimeter metal tube (13) preferably divided into sections connected one to the other in such a way as to adapt to the increasing perimeter development during the phases of tensioning of the layer (10) and comprising a perimeter flap (15) configured to adapt to the edge of the membrane (11).

10. Floating cover system (1) according to the preceding claim, **characterised in that** said flap, with hydraulic trap function, (15) comprises one or more pockets suitable for accommodating a ballast, preferably made up of sand.

11. Floating cover system (1) according to one or more of the preceding claims, **characterised in that** said floating raft (20) further comprises a plurality of rigid connectors (25) suitable for transferring the loads to the floating raft (20) itself, said connectors (25) preferably comprising in turn a box element (25a) configured and dimensioned to be inserted in the free space at the joining point (22) between the modular floats (21) in such a way as to occupy the free space between the modular floats (21) at the joining points (22) and a tubular element (25b) configured to be inserted at the joining space (22), said connectors (25) being suitable for connecting said floating raft (20) to said one or more support elements (50) and to said cover layer (10) by means of said tensioning system (12).

12. Floating cover system (1) according to one or more of the preceding claims, **characterised in that** it further comprises a biogas/emissions catchment system in turn comprising at least one extraction pipe (80) connected to at least one of said support element (50) at a suitable flange (52) for connection provided at the drainage system of said support element (50), said catchment system further comprises at least one drum (90) for the winding of said extraction pipe (80) and at least one support device (91) of the extraction pipe comprising a vertically movable head (92) suitable for supporting the upper end of said extraction pipe (80) and for moving the vertical axis to the high point (81) of said pipe (80) in such a way as to maintain the height of said high point (81) always above the height of the last coil (80a) wound on said drum (90), so as to ensure the outflow of any condensation.

13. Floating cover system (1) according to one or more of the preceding claims, **characterised in that** it provides measuring probes, independent of external environmental conditions, which allow optimal aspiration of volatile compounds potentially harmful to the environment, and removal of rainwater.

Fig. 1

Fig. 2

Fig. 3

Fig. 4

Fig. 5

EP 4 317 088 A1

Fig. 6

Fig. 7

Fig. 8

Fig. 9

Fig. 10

Fig. 11

25a

25b

26

## Fig. 12

50

51

59

40

59

## Fig. 13

Fig. 14

Fig. 15

Fig. 16

Fig. 17

Fig. 18

Fig. 19

Fig. 20

Fig. 21

Fig. 22

Fig. 23

Europäisches
Patentamt

European
Patent Office

Office européen
des brevets

# EUROPEAN SEARCH REPORT

Application Number

EP 23 18 7432

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CA 2 252 294 A1 (DEGARIE CLAUDE J [CA]) 30 April 2000 (2000-04-30) * figures 1-9 * * pages 2-9 * | 1-13 | INV. C02F3/28 C12M1/107 B65D88/34 |
| X | EP 2 966 963 B1 (ENGEL RÉMY MARIE ARNAUD [FR]; MONCORGER JEOFFREY JEAN RAYMOND [FR]) 29 March 2017 (2017-03-29) * figures 1,2 * * paragraphs [0020] – [0029] * | 1-13 | |
| X | IT CR20 010 010 A1 (ECOMEMBRANE SRL [IT]) 13 June 2003 (2003-06-13) | 1 | |
| A | * page 1; figures 1, 14 * | 2-13 | |

TECHNICAL FIELDS
SEARCHED (IPC)

C02F
C05G
C12M
C05F
B65D

The present search report has been drawn up for all claims

| Place of search | Date of completion of the search | Examiner |
|---|---|---|
| The Hague | 25 September 2023 | Onel Inda, Santiago |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 18 7432

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2023

| Patent document cited in search report | | Publication date | Patent family member(s) | | Publication date |
|---|---|---|---|---|---|
| CA 2252294 | A1 | 30-04-2000 | NONE | | |
| EP 2966963 | B1 | 29-03-2017 | EP | 2966963 A1 | 20-01-2016 |
| | | | FR | 3000639 A1 | 11-07-2014 |
| | | | WO | 2014108624 A1 | 17-07-2014 |
| IT CR20010010 | A1 | 13-06-2003 | NONE | | |

EPO FORM P0459

For more details about this annex : see Official Journal of the European Patent Office, No. 12/82